# EUROPEAN PATENT SPECIFICATION

(11) **EP 1 643 864 B1**
(45) Date of publication and mention of the grant of the patent: **10.08.2011**
(21) Application number: 04740233.4
(22) Date of filing: 24.06.2004
(51) Int. Cl.: A23L 1/302, A23L 1/304, A23L 1/30, A61K 33/26, A61K 33/30

(54) **AIDING OF COGNITIVE FUNCTION**
UNTERSTÜTZUNG DER KOGNITIVEN FUNKTION
AIDE A LA FONCTION COGNITIVE

(30) Priority: 21.07.2003 EP 03077270
(43) Date of publication of application: 12.04.2006
(60) Divisional application: 06117650.9
(73) Proprietor: Unilever N.V., 3013 AL Rotterdam (NL); Unilever PLC, London EC4Y 0DY (GB)
(72) Inventor: Van Klinken, Bernardus Jan-Willem Unilever R & D, 3133 AT Vlaardingen (NL); Bryan, Janet Consumer Science Program, Adelaide BC, S.A. 5000 (AU)
(74) Representative: Wurfbain, Gilles L.
(86) International application number: PCT/EP2004/006817
(87) International publication number: WO 2005/013724

(56) References cited:
- WO-A-00/62812
- WO-A-03/003981
- US-A1- 2002 045 660
- US-A1- 2003 044 472
- DATABASE WPI Week 1997 Derwent Publications Ltd., London, GB; AN 1997-213445 XP002292286 GAO C, HUANG T, ZHANG W: "Nutritious oral liq. contg. natural ingredients. vitamins and trace elements, promotes bone growth and brain development" & CN 1 097 607 A (MEDICAL INST 999 ENTERPRISE GROUP) 25 January 1995 (1995-01-25)
- DATABASE WPI Week 1997 Derwent Publications Ltd., London, GB; AN 1997-458010 XP002292287 CHEN W, JIANG X, LIU J: "Nutritious soy sauce and its production" & CN 1 117 816 A (CHEN W) 6 March 1996 (1996-03-06)
- DATABASE WPI Week 2003 Derwent Publications Ltd., London, GB; AN 2003-514435 XP002292288 ZHAO G: "Production method of coated and shaped tablet health-care food with the functions of increasing body height and reducing weight for youngsters" & CN 1 411 747 A (ZHAO G) 23 April 2003 (2003-04-23)
- NEUMANN C, HARRIS DM, ROGERS LM: "Contribution of animal source foods in improving diet quality and function in children of the developping world" NUTRITION RESEARCH, vol. 22, 2002, pages 193-220, XP002257664
- DATABASE BIOSIS [Online] BIOSCIENCES INFORMATION SERVICE, PHILADELPHIA, PA, US; SESHADRI S, HIRODE K, NAIK P, MALHOTRA S: "Behavorial responses of young anemic Indian children to iron folic-acid supplements" XP002257665 retrieved from BIOSIS Database accession no. PREV198375074283
- KOLETZKO B ET AL: "GROWTH, DEVELOPMENT AND DIFFERENTIATION: A FUNCTIONAL FOOD SCIENCE APPROACH" BRITISH JOURNAL OF NUTRITION, CAMBRIDGE, GB, vol. 80, no. SUPPL 1, 1998, pages S05-S45, XP009027350

## Description

### FIELD OF THE INVENTION

The present invention relates to the use of certain micronutrients in the preparation of an edible composition for aiding the cognitive development or performance of humans having an age of up to 18 years. It also relates to a method of aiding the cognitive development or performance of such humans by using the aforementioned edible composition.

### BACKGROUND OF THE INVENTION

In recent years there has been an increasing interest in the link between nutrition and cognitive performance in humans, especially, in the impact of nutrition on the development of cognitive abilities during childhood.

Iron is thought to be an important nutrient for the cognitive performance and development of children because the areas of the brain that are sensitive to iron deficiency (such as the cortex, frontal lobes and hippocampus) are the areas responsible for higher level cognitive functions such as executive functions and memory (Beard, 1995, American Journal of Nutrition, 62, 709-710).

Zinc may have independent effects on cognitive performance, as well as an indirect effect through the enhancement of the efficacy of iron (Black & Miguel, 2001, The emerging roles of zinc in human nutrition, development and infectious disease, part 1, Nutrition Today, 36, 281-290). Zinc is important for cognitive performance and development of children because it is important for myelination and release of the neurotransmitters GABA and glutamate that are key modulators of neuronal excitability (Bhatnagar & Taneja, 2001, Zinc & cognitive development. British Journal of Nutrition, 85, S139-S145). Intervention studies show that 20mg/day zinc plus other micronutrients has a positive effect on reasoning and visual recognition in Chinese and Mexican American school-aged children (Penland, 2000, Behavioural data and methodological issues in studies of zinc nutrition in humans, Journal of Nutrition, 130, 361S-364S).

Vitamin B-6 may affect the glutaminergic system and N-methyl-D-aspartate (NMDA) receptor that is important for learning and memory (Calvaresi & Bryan, 2001, B vitamins, cognition and ageing: A review. Journal of Gerontology: Psychological Sciences, 56, P327-P339). Correlational studies in children in India (Refsum et al., 2001 - Hyperhomocysteinemia and elevated methylmalonic acid indicate a high prevalence of cobalamin deficiency in Asian Indians. American Journal of Clinical Nutrition, 74, 233-241) and Indonesia (Setiawan, et al., 2000 Vitamin B-6 inadequacy is prevalent in rural, and urban Indonesian children. Journal of Nutrition, 130, 553-558) have shown associations between B-12 and B-6 deficiency and poorer cognitive performance.

Certain omega-3 Polyunsaturated Fatty Acids (PUFAs) have been linked with cognitive performance. Studies have shown that infants who are fed formula milk supplemented with docosahexaenoic acid (DHA), α-linolenic acid (ALA) and arachidonic acid (AA), and those who are breast-fed demonstrate better visual acuity and cognitive performance, including problem solving (Uauy et al, 2001, Essential fatty acids in somatic growth and brain development. Nutrition and fitness: Diet, genes, physical activity and health. NY: Karger. pp.134-160).

WO03/003981 discloses compositions which are claimed to be beneficial in improving mental performance. These compositions comprise vitamin B12 on ion exchange resin, docosahexaenoic acid, certain herbal extracts and may comprise iron and/or zinc.

WO00/62812 discloses nutritional compositions which are claimed to be beneficial for the improvement of cognitive performance. The composition comprise caffeine, choline, gamma aminobutyric acid, L-phenylalanine and taurine.

US2003/0044472 A1 discloses compositions for treating Attention Deficit/Hyperactivity Disorder. The compositions comprise omega 3-fatty acids, certain B vitamins and zinc.

US 2002/0045660 A1 discloses infant milk formulae comprising PDA. Supplementation of infant formula milk with DHA is said to enhance neurological development of a pre-term infant.

Neumann et al (2002, contribution of animal source foods in improving diet quality and functions in children in the developing world, Nutrition Research, 22, 193-220) review the effect at iron, zinc, vitamins B12 and A on child growth, cognitive development and health.

Abstract No. XP-002257665, Behavioral Responses of Young Anemic Indian Children to Iron Folic Acid Supplements discloses the results of supplementing young anemic Indian children with iron and folic acid supplements. The supplements are reported to raise Hb levels and to provide improved intelligence test results, particularly in the performance section.

There is an increasing demand in the art for edible compositions that will aid the cognitive development or performance of humans, especially those having an age of up to 18 years. Preferably, such edible compositions can be added to ordinary foodstuffs, or, they can be consumed separately e.g. as a foodstuff in its own right.

The present invention seeks to provide edible compositions that aid the cognitive development and/or performance of humans, especially those having an age of up to 18 years.

In particular, it is an object of the invention to provide compositions that aid the cognitive development and/or performance of humans, especially those having an age of up to 18 years and that can be added to ordinary foodstuffs, or, can be consumed separately.

### SUMMARY OF THE INVENTION

Surprisingly, it has now been found that edible compositions according to the invention, can aid the cognitive development or performance of humans, especially those having an age of up to 18 years. These edible compositions can be added to ordinary foodstuffs, or, can be consumed separately.

According to a first aspect, the present invention provides the use of;
1. a bioavailable iron compound and a bioavailable zinc compound in a weight ratio of from 2:1 to 5:1, and
2. at least one B vitamin,
in the manufacture of an edible composition, wherein said composition comprises 0.5 to 30 mg bioavailable iron per serving of the edible composition, for use in aiding the cognitive development or cognitive performance of humans having an age of up to 18 years.

It is preferred according to both the second and first aspect of the invention that the edible composition after consumption aids the cognitive development or performance of humans having an age of from 6 to 9 years.

The terms "cognition" and "cognitive function" as used herein are interchangeable and refer to a construct of a range of functions:
- Intelligence (fluid, crystallised),
- Creativity (generate and open to new ideas, perceive new relationships),
- Memory (encoding, storage, retrieval),
- Executive functions (dealing with novelty, planning & implementation, monitoring performance, vigilance, inhibition of task irrelevant info),
- Cognitive resources (speed of information processing, working memory capacity, attentional capacity).

The term "cognitive development" as used herein refers to the development of cognition (cognitive function) over time. The quality of cognition (cognitive function) in a human can be assessed by tests measuring cognitive performance. As cognition (cognitive function) of a growing child develops over time with brain development, measuring the cognitive performance over time for a given child, provides a measure of cognitive development. For example, for children aged 6-9 years, cognitive development is generally recognised by :
- A spurt in information processing capacity and/or executive functions.
- Development of the frontal lobes of the brain (involved in executive functions). These growth spurts may also occur at other periods during childhood (e.g. 0-2 and mid-teens).

The present invention provides a convenient and effective way of aiding the cognitive development or performance of humans having an age of up to 18 years.

All amounts are as percentages by weight unless otherwise stated. For the edible compositions, all percentages are by weight based on the total weight of the composition unless otherwise stated.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

A serving of the edible composition as referred to herein refers to the amount of the edible composition that is provided to be consumed as a single portion, typically in a single sitting. For beverages, the typical serving size is in the range of from 100 to 500 ml and for a bar product or cookie etc will be in the range of from 20 to 100 g. However, it will be understood that the serving size will vary according to the type of edible composition.

### DETAILED DESCRIPTION

According to the present invention, edible compositions that comprise the specific mixture of nutrients according to the present invention can be used to aid the cognitive development or performance of humans having an age of up to 18 years. Further details of the edible compositions and their components are given below.

### Iron compound

The present invention uses a bioavailable iron compound, that is, an iron compound that is at least partially bioavailable to the body. Any bioavailable iron compound may be used according to the present invention although ferrous iron is generally better utilized by the body than ferric iron.

Bioavailable iron compounds which can be used include edible inorganic iron compounds such as edible ferrous compounds, for example ferrous sulfate. Whilst ferrous iron is typically more bioavailable, certain ferric salts can also provide highly bioavailable compounds of iron and these may also be used according to the present invention. Suitable examples include ferric sulfate and ferric chloride and mixtures thereof.

Edible bioavailable iron organic compounds may also be used according to the present invention. Preferred examples include ferrous carboxylate salts such as ferrous fumurate, ferrous succinate, ferrous tartarate, ferrous lactate, ferrous gluconate, ferrous citrate and mixtures thereof. Whilst, as stated above ferrous iron is typically more bioavailable, highly bioavailable edible ferric compounds that may be used in the present invention include ferric carboxylate salts such as ferric ammonium citrate, ferric citrate and mixtures thereof.

An especially preferred bioavailable iron organic compound is ferric EDTA (sodium ferric ethylenediaminetetraacetic acid or sodium iron ethylenediaminotetracetic acid, NaFeEDTA). Ferric EDTA has the advantage that it does not appreciably interchange with other cations often present in a edible compositions with added vitamin/mineral mixes (e.g., sodium, calcium, potassium, zinc, iodine, vitamin C, vitamin E, and the like) and thus no significant free iron is generated. This aids in the colour and flavour stability of the food product. It is also relatively stable in edible compositions and has good bioavailability.

Other edible iron organic compounds which can be used according to the present invention include bioavailable iron-sugar-carboxylate complexes. These materials are referred to herein as "complexes," but they may, in fact, exist in solution as complicated, highly hydrated, protected colloids. However, the term "complex" is used for the purpose of simplicity. Such materials are described in US 6 509 045.

In these iron-sugar-carboxylate complexes, the carboxylate provides the counterion for the ferrous (preferred) or ferric iron. Sugars that can be used to produce these complexes include any of the edible saccharidic sugars such as glucose, sucrose and fructose, mannose, galactose, lactose, maltose, and the like, with sucrose and fructose being preferred. Mixtures thereof may also be used. The carboxylic acid used may be any edible carboxylic acid such as citric acid, malic acid, tartaric acid, lactic acid, succinic acid, propionic acid, and mixtures thereof. Particular examples include ferric saccharide, ferric saccharate and mixtures thereof.

US 6 509 045 also discloses that it is known that iron is more bioavailable if administered in the form of chelates wherein the chelating ligands are amino acids or protein hydrolysates (see U.S. 3,969,540 and U.S. 4,020,158). These chelated iron compounds are known in the art by various names such as iron proteinates, iron amino acid chelates and peptide or polypeptide chelates. Such materials may also be used according to the present invention.

Particularly suitable ferrous amino acid chelates are those where the reacting ligands are glycine, lysine, and leucine, including the ferrous amino acid chelate where the reacting ligand is glycine.

Ferrous amino acid chelates particularly suitable as highly bioavailable amino acid chelated irons for use in the present invention are those having a ligand to metal ratio of at least 2:1. For example, suitable ferrous amino acid chelates having a ligand to metal mole ratio of two are those of formula: Fe(L) 2 where L is an alpha amino acid, dipeptide, tripeptide or quadrapeptide reacting ligand. Thus, L can be any reacting ligand that is a naturally occurring alpha amino acid selected from alanine, arginine, asparagine, aspartic acid, cysteine, cystine, glutamine, glutamic acid, glycine, histidine, hydroxyproline, isoleucine, leucine, lysine, methionine, omithine, phenylalanine, proline, serine, threonine, tryptophan, tyrosine and valine or dipeptides, tripeptides or quadrapeptides formed by any combination of these alpha amino acids. See U.S. Pat. No. 3,969,540 and U.S. Pat. No. 4,020,158.

Elemental iron may also be used according to the present invention and is considered herein to be a bioavailable compound even though, strictly speaking, it is not a compound.

Mixtures of one or more of any of the above bioavailable iron compounds may also be used. It is preferred that the bioavailable iron compound is selected from ferrous inorganic compounds, ferrous carboxylate salts, iron-sugar-carboxylate complexes, chelated iron compounds and mixtures thereof. The bioavailable iron compounds, where appropriate, may be in a hydrated form. An especially preferred bioavailable iron compound is sodium ferric ethylenediaminetetraacetic acid or sodium iron ethylenediaminotetracetic acid, known as NaFeEDTA.

The bioavailable iron compounds, may if desired, be in an encapsulated form.

The amount of bioavailable iron compound used in the edible composition may be any amount which aids the cognitive development or performance of humans having an age of up to 18 years provided that the requirement for the weight ratio of iron to zinc according to the present invention is met. It is preferred that an amount of up to 50mg of iron from the bioavailable iron compound is used in the edible compositions, more preferably 0.5 to 30 mg, most preferably 1 to 20 mg, such as 2 to 15mg per serving of the edible composition. These amounts are based on the weight of the iron in the bioavailable iron compound and not on the total weight of the bioavailable iron compound. Thus the weight of the bioavailable iron compound will be correspondingly greater depending upon the compound used.

### Zinc Compounds

The present invention uses a bioavailable zinc compound, that is, a zinc compound that is at least partially bioavailable to the body.

Bioavailable zinc compounds which can be used include edible inorganic zinc compounds such as zinc oxide, zinc sulfate, zinc chloride and mixtures thereof.

Edible bioavailable zinc organic compounds may also be used according to the present invention. Preferred examples include zinc carboxylate salts such as zinc acetate, zinc gluconate, zinc ascorbate, zinc citrate, zinc aspartate, zinc picolinate and mixtures thereof.

Amino acid chelated zinc compounds of the typed referred to above for iron compounds may also be used.

Mixtures of one or more of any of the above bioavailable zinc compounds may also be used. Preferably the bioavailable zinc compound is selected from inorganic zinc salts, zinc carboxylate salts, chelated zinc compounds and mixtures thereof. The compounds, where appropriate, may be in a hydrated form.

The bioavailable zinc compounds, may if desired, be in an encapsulated form.

According to one embodiment of the invention, it is particularly preferred that iron compound comprises NaFeEDTA and the zinc compound comprises zinc sulphate.7H₂O.

The amount of bioavailable zinc compound used in the edible composition may be any amount which aids the cognitive development or performance of humans having an age of up to 18 years provided that the requirement for the weight ratio of iron to zinc according to the present invention is met. It is preferred that an amount of up to 50 mg of zinc from the bioavailable zinc compound is used in the edible compositions, more preferably 0.5 to 20 mg, most preferably 0.7 to 15 mg, such as 1 to 10 mg per serving of the food product. These amounts are based on the weight of the zinc in the bioavailable zinc compound and not on the total weight of the bioavailable zinc compound. Thus the weight of the bioavailable zinc compound will be correspondingly greater depending upon the compound used.

The weight ratio of the bioavailable iron compound to the bioavailable zinc compound is in the range of from 2:1 to 5:1, most preferably 2:1 to 4:1.

### B vitamins

The present invention uses at least one B vitamin, preferably, selected from vitamins B6, B11 and B12. It is especially preferred that the edible compositions comprises any two of vitamins B6, B11 and B12, even more preferably each of B6, B11 and B12.

The at least one B vitamin is preferably used in amounts of up to 5 mg in a serving of the edible composition, preferably 0.05 to 3 mg, more preferably 0.1 to 2mg.

It is preferred that Vitamins B6 and B11 are individually used in amounts of from 0.05 to 5 mg, more preferably from 0.075 to 3 mg, most preferably from 0.1 to 2 mg per serving of the edible composition.

It is preferred that Vitamin B12 is used in amounts of from 0.0001 to 1 mg, preferably from 0.001 to 0.5 mg, most preferably from 0.0015 to 0.2 mg per serving of the edible composition.

For all the B vitamins above, the amount of the ingredient referred to is to the amount of the active ingredient.

### Polyunsaturated fatty acid

The present invention preferably further uses a polyunsaturated fatty acid in the preparation of the edible composition. Preferably, any oil comprising omega 3 fatty acids, especially docosahexaenoic acid (DHA, C20:5) and/or eicosapentaenoic acid (EPA, C22:5) may be used. Preferred omega 3 fatty acids are the following C18:3, C18:4, C20:4, C20:5, C22:5 and C22:6.

Preferably the polyunsaturated fatty acid may be incorporated by the inclusion in the edible composition of fish oil and/or an oil derived from algae.

The polyunsaturated fatty acid preferably comprises DHA and EPA in a weight ratio of at least 2:1, more preferably at least 3:1, such as at least 4:1.

The polyunsaturated fatty acid is preferably used in amounts of up to 2g per serving of the edible composition, more preferably 50 mg to 1 g per serving, more preferably 100 to 500 mg per serving.

It is especially preferred according to the present invention that the polyunsaturated fatty acid is used with either a bioavailable iron compound or a bioavailable zinc compound in the edible composition. Most preferred is that a polyunsaturated fatty acid, especially a mixture of DHA and EPA is used with both a bioavailable iron compound and a bioavailable zinc compound in the edible composition.

### Vitamins A and C

It known that concurrent supplementation with vitamin C can improve iron absorption by the body. Vitamin A may enhance iron mobilisation and vitamin A and beta carotene increase the absorption of non-heme iron (Mejia & Chew, 1988, Hematological effects of supplementing anaemic children with vitamin A alone and in combination with iron. American Journal of Clinical Nutrition, 48, 1271-1276).

Vitamin A and/or vitamin are C preferably included in the edible composition.

Vitamin A is preferably used in amounts of from 0.5 to 50 mg, preferably 1 to 20 mg, such as 2 to 10 mg based on the retinol acetate equivalent amount. Vitamin C is preferably used in amounts of from 1 to 100 mg, preferably 5 to 75 mg, such as 10 to 65 mg.

It is especially preferred according to the present invention that the edible composition comprises in addition to the B vitamins recited herein a polyunsaturated fatty acid, a bioavailable iron compound, a bioavailable zinc compound and vitamins A and/or C, especially both vitamins A and C.

### Types of edible composition

The edible composition of the invention may be in the form of a nutritional supplement (such as a tablet, powder, capsule or liquid product) or in the form of a food composition (product) including a beverage. Food compositions are especially preferred according to the present invention.

It is preferred that the edible composition is a food composition which further comprises at least one of protein, carbohydrate or fat. It is especially preferred that the food composition comprises at least protein and carbohydrate, most especially in the amounts given hereinbelow. Alternatively, a source of one of protein, carbohydrate and/or fat can be given as the same time as the edible composition, for example, if the edible composition does not contain substantial amounts of these ingredients. In this case, the edible composition of the invention and an additional food product would provide a kit that could be used as according to the present invention. The comments herein to the protein, carbohydrate and fat content etc apply equally to the additional food product in such a kit.

The edible composition may be for example; a solid product, a powdered product, a liquid, a flowable, spoonable, pourable or spreadable product or a bar etc. The edible composition may be a powder which is mixed with a liquid, such as water or milk, to produce a liquid or slurry product.

The edible compositions may be suitably selected from dairy based products (such as milk based products and drinks), soy based products including drinks, breads and cereal based products (including pasta or noodle based products and cereal bars), cakes, biscuits, ice creams, desserts, soups, porridge-type products, beverages including a ready-to-drink liquid, a liquid produced from a soluble powdered product, bar products, confectionery, snack foods, ready-to-eat meal products, prepacked meal products, and dried meal products etc and soluble powdered products.

It is especially preferred that the edible compositions are in the form of a beverage (including dairy and soy based beverages), a bar product, a porridge, a biscuit or a cake.

Where an edible powder is provided according to the invention, this will be usually added to another food product, such as a beverage or a biscuit, to make consumption more convenient.

### Protein

The edible composition further preferably comprises protein, this is preferred to provide energy to the consumer of the edible food product as well as the specific micronutrients described hereinabove. Any suitable protein may be used depending upon the type of edible composition.

Suitable sources of protein include dairy sources such as milk, yoghurt, kefir, cheese or cream. Other animal sources may also be used depending upon the type of edible composition. Alternatively the edible composition may comprise vegetable derived protein such as soy-protein, rice protein, pea protein or wheat protein.

The amount of protein in the edible composition is preferably an amount of from 0.1 to 30 or 40% by weight of the edible composition, preferably 0.5 to 25%wt, most preferably 1 to 20 %wt.

### Carbohydrate

The edible compositions may also comprise one or more carbohydrates, preferably in an amount of from 1 to 90% by weight based on the weight of the composition, more preferably of from 5 to 80%wt, most preferably 10 to 75%wt. This is preferred to provide energy to the consumer of the edible food product as well as the specific micronutrients described hereinabove.

Any suitable carbohydrate may be used, for example sucrose, lactose, glucose, fructose, corn syrup, maltodextrins, starch, modified starch or mixtures thereof.

The edible composition may also comprise dietary fibres, for example in an amount of from 0.1 to 10% by weight based on the weight of the composition, preferably 0.5 to 5wt.

It is especially preferred that the edible composition comprises both protein and carbohydrate, preferably in the amounts given above.

### Fat

The edible composition may comprise edible fats in addition to any polyunsaturated fatty acid referred to above. The compositions preferably comprise an amount of up to 40% by weight based on the weight of the composition of other fats, more preferably from 0.5 to 30 or 35%wt, most preferably from 0.75 to 10 or 20% fat.

Any suitable fat may be used with vegetable fats being especially preferred. Suitable examples include vegetable fats, plant oils, nut oils, seed oils, or mixtures thereof. Saturated or unsaturated (mono-unsaturated and poly-unsaturated) fats may be used, although saturated fats are less preferred.

The amounts of protein, fat, carbohydrate and other ingredients in the edible composition will vary according to the product format of the composition and also, where required, according to national or regional legislation.

### Optional ingredients

The edible composition may optionally comprise fruit or vegetable pieces or particles, concentrates, juice or puree, preferably in amounts of up to 60% by weight of based on the weight of the edible composition. Preferably the compositions comprise 0.1 to 40%wt, more preferably 1 to 20%wt of these ingredients. The amount of these ingredients will depend upon the type of edible composition.

The edible composition may also comprise 0.1 to 15% by weight of edible salts based on the weight of the composition, preferably 3 to 8%wt. Any edible salts may be used, for example, sodium chloride, potassium chloride, alkali metal or alkaline earth metal salts of citric acid, lactic acid, benzoic acid, ascorbic acid, or, mixtures thereof. Calcium salts may also be used such as calcium chloride and calcium caseinate. The edible composition may further comprise ingredients selected from other added vitamins and/or minerals, herbs, spices, flavourings, stabilisers, emulsifiers, aromas, antioxidants, colourants, preservatives or mixtures thereof. These ingredients are used in conventional amounts.

These other added vitamins and/or minerals are preferably selected from at least one of vitamins D, E, H, K and calcium, magnesium, potassium, iodine, manganese, molybdenum, phosphorus, selenium and/or chromium.

### Preparation

The edible compositions of the invention may be prepared by any suitable method of preparation, including mixing, blending, homogenising, high-pressure homogenising, emulsifying, dispersing, and/or encapsulating depending upon the type of edible composition. It is well within the skill of the person skilled in the art to select the most appropriate preparation method for different types of edible compositions.

### Method of aiding the cognitive development or performance

The invention also relates to the aforementioned method for aiding the cognitive development or performance of humans having an age of up to 18 years according to the second aspect of the invention.

The total effective administered to a subject according to the invention may vary according to the needs of the person to whom it is administered, e.g. age, general nutrition status etc. Typical amounts administered by the administration of the edible composition for each of the micronutrients according to the invention are listed above under the heading referring to that micronutrient. These are the typical amounts administered per day per micronutrient. An effective daily amount may be administered by a single dose or by multiple doses.

The consumption of the edible composition of the invention preferably occurs as a part of the daily diet. It is preferred that the edible composition is consumed daily.

The invention is further described by way of the following examples that are to be understood as not limiting. Further examples within the scope of the invention will be apparent to the person skilled in the art.

### EXAMPLES

### Example 1 - beverage

5 g of a powder having the composition shown in table 1 was added to 100 ml of a commercially available aqueous soy-based drink (ADES soy based fruit drink) comprising 0.6% wt of soy protein. The resulting beverage was mixed until the powder had dissolved.

**Table 1 - composition of the powder**

| Ingredient | Amount of compound | Amount of active ingredient |
|---|---|---|
| FeNaEDTA | 84 mg | 10 mg iron |
| ZnSO₄.7H₂0 | 23.1 mg | 5 mg zinc |
| Fish oil powder *1 | 763 mg | 88 mg DHA and 22 mg EPA |
| Vitamin B6 (pyridoxine.HCl) | 1.34 mg | 1 mg |
| Vitamin B11 (folic acid) | 0.17 mg | 0.15 mg |
| Vitamin B12 (cyanocobalamin, 0.1 % WS) | 1.65 mg | 0.0015 mg |
| Vitamin A (retinol acetate dry, 325.000 IU/g) | 4.51 mg | 0.4 mg (as retinol equivalent) |
| Vitamin C (Ascorbic acid) | 54 mg | 54 mg |
| Maltodextrin | 2640 mg | 2640 mg |
| Lecithin | 300 mg | 300 mg |
| Citric acid | 40 mg | 40 mg |
| Powdered sugar | 1067 mg | 1067 mg |
| Flavours | 20 mg | 20 mg |

| | | |
|---|---|---|
| *1 - the fish oil powder contained 20% wt in total of DHA/EPA in a weight ratio of 4.5:1. | | |

The edible composition can be consumed by humans aged up to 18 years of age to aids cognitive development or performance.

## Claims

1. The use of:
1. a bioavailable iron compound and a bioavailable zinc compound in a weight ratio of from 2:1 to 5:1 and
2. at least one B vitamin,
in the manufacture of an edible composition, said composition comprising 0.5 to 30 mg bioavailable iron per serving of the edible composition for use in aiding the cognitive development or cognitive performance of humans having an age of up to 18 years.

2. The use according to claim 1, wherein the bioavailable iron compound is selected from ferrous inorganic compounds, ferrous carboxylate salts, iron-sugar-carboxylate complexes, chelated iron compounds and mixtures thereof.

3. The use according to any one of the preceding claims, wherein the edible composition comprises 0.5 to 20 mg per serving of the edible composition of zinc from the bioavailable zinc compound.

4. The use according to any one of the preceding claims, wherein the bioavailable zinc compound is selected from inorganic zinc salts, zinc carboxylate salts, chelated zinc compounds and mixtures thereof.

5. The use according to any one of the preceding claims, wherein the wherein the weight ratio of the bioavailable iron compound to the bioavailable zinc compound is in the range of from 2:1 to 4:1.

6. The use according to any one of the preceding claims, wherein the edible composition further comprises at least one polyunsaturated fatty acid.

7. The use according to claim 6, wherein the edible composition comprises up to 2 g per serving of the polyunsaturated fatty acid.

8. The use according to either one of claims 6 or 7, wherein the polyunsaturated fatty acid comprises docosahexaenoic acid and eicosapentaenoic acid in a weight ratio of at least 2:1.

9. The use according to any one of the preceding claims, wherein the at least one B vitamin is selected from vitamins B6, B11 and B12.

10. The use according to any one of the preceding claims, wherein the edible composition further comprises vitamin A and/or vitamin C.

11. The use according to any one of the preceding claims, wherein the edible composition is a food composition.

12. The use according to any one of the preceding claims, wherein the food composition further comprises protein and/or carbohydrate.

## Patentansprüche

1. Verwendung:
1. einer bioverfügbaren Eisenverbindung und einer bioverfügbaren Zinkverbindung in einem Gewichtsverhältnis von 2:1 bis 5:1 und
2. wenigstens eines B-Vitamins,
bei der Herstellung einer essbaren Zusammensetzung, wobei die Zusammensetzung 0,5 bis 30 mg bioverfügbares Eisen pro Portion der essbaren Zusammensetzung umfasst, zur Verwendung bei der Unterstützung der kognitiven Entwicklung oder der kognitiven Leistungsfähigkeit von Menschen, die ein Alter von bis zu 18 Jahren haben.

2. Verwendung gemäß Anspruch 1, wobei die bioverfügbare Eisenverbindung aus anorganischen Eisen(II)-Verbindungen, Eisen(II)-carboxylatsalzen, Eisen-Zucker-carboxylatkomplexen, chelatisierten Eisenverbindungen und Gemischen davon ausgewählt ist.

3. Verwendung gemäß einem der vorangehenden Ansprüche, wobei die essbare Zusammensetzung 0,5 bis 20 mg pro Portion der essbaren Zusammensetzung an Zink aus der bioverfügbaren Zinkverbindung umfasst.

4. Verwendung gemäß einem der vorangehenden Ansprüche, wobei die bioverfügbare Zinkverbindung aus anorganischen Zinksalzen, Zink-carboxylatsalzen, chelatisierten Zinkverbindungen und Gemischen davon ausgewählt ist.

5. Verwendung gemäß einem der vorangehenden Ansprüche, wobei das Gewichtsverhältnis der bioverfügbaren Eisenverbindung zu der bioverfügbaren Zinkverbindung im Bereich von 2:1 bis 4:1 liegt.

6. Verwendung gemäß einem der vorangehenden Ansprüche, wobei die essbare Zusammensetzung außerdem wenigstens eine mehrfach ungesättigte Fettsäure umfasst.

7. Verwendung gemäß Anspruch 6, wobei die essbare Zusammensetzung bis zu 2 g der mehrfach ungesättigten Fettsäure pro Portion umfasst.

8. Verwendung gemäß einem der Ansprüche 6 oder 7, wobei die mehrfach ungesättigte Fettsäure Docosahexaensäure und Eicosapentaensäure in einem Gewichtsverhältnis von weniger 2:1 umfasst.

9. Verwendung gemäß einem der vorangehenden Ansprüche, wobei das wenigstens eine B-Vitamin aus den Vitaminen B6, B11 und B12 ausgewählt ist.

10. Verwendung gemäß einem der vorangehenden Ansprüche, wobei die essbare Zusammensetzung außerdem Vitamin A und/oder Vitamin C umfasst.

11. Verwendung gemäß einem der vorangehenden Ansprüche, wobei die essbare Zusammensetzung eine Nahrungsmittelzusammensetzung ist.

12. Verwendung gemäß einem der vorangehenden Ansprüche, wobei die Nahrungsmittelzusammensetzung außerdem Protein und/oder Kohlenhydrat umfasst.

## Revendications

1. Utilisation :
1. d'un composé de fer biodisponible et d'un composé de zinc biodisponible dans un rapport en poids de 2 : 1 à 5 : 1 et
2. d'au moins une vitamine B,
dans la fabrication d'une composition comestible, ladite composition comprenant 0,5 à 30 mg de fer biodisponible par ration de composition comestible, à utiliser dans l'aide au développement cognitif ou à la performance cognitive d'êtres humains ayant un âge allant jusqu'à 18 ans.

2. Utilisation selon la revendication 1, dans laquelle le composé de fer biodisponible est choisi parmi les composés inorganiques ferreux, les sels de carboxylate ferreux, les complexes fer-sucre-carboxylate, les composés de fer chélaté, et leurs mélanges.

3. Utilisation selon l'une quelconque des revendications précédentes, dans laquelle la composition comestible comprend 0,5 à 20 mg de zinc provenant du composé de zinc biodisponible par ration de composition comestible.

4. Utilisation selon l'une quelconque des revendications précédentes, dans laquelle le composé de zinc biodisponible est choisi parmi les sels de zinc inorganiques, les sels de carboxylate de zinc, des composés de zinc chélaté et leurs mélanges.

5. Utilisation selon l'une quelconque des revendications précédentes, dans laquelle le rapport en poids entre le composé de fer biodisponible et le composé de zinc biodisponible est dans la plage de 2 : 1 à 4 : 1.

6. Utilisation selon l'une quelconque des revendications précédentes, dans laquelle la composition comestible comprend en outre au moins un acide gras polyinsaturé.

7. Utilisation selon la revendication 6, dans laquelle la composition comestible comprend jusqu'à 2 g par ration d'acide gras polyinsaturé.

8. Utilisation selon l'une quelconque des revendications 6 ou 7, dans laquelle l'acide gras polyinsaturé comprend de l'acide docosahexaénoïque et de l'acide eicosapentaénoïque dans un rapport en poids d'au moins 2 : 1.

9. Utilisation selon l'une quelconque des revendications précédentes, dans laquelle la au moins une vitamine B est choisie parmi les vitamines B6, B11 et B12.

10. Utilisation selon l'une quelconque des revendications précédentes, dans laquelle la composition comestible comprend en outre de la vitamine A et/ou de la vitamine C.

11. Utilisation selon l'une quelconque des revendications précédentes, dans laquelle la composition comestible est une composition alimentaire.

12. Utilisation selon l'une quelconque des revendications précédentes, dans laquelle la composition alimentaire comprend en outre des protéines et/ou glucides.
